(19) **Europäisches Patentamt / European Patent Office / Office européen des brevets**

(11) **EP 4 159 208 A1**

(12) # EUROPEAN PATENT APPLICATION

(43) Date of publication:
05.04.2023 Bulletin 2023/14

(21) Application number: 21200432.9

(22) Date of filing: 01.10.2021

(51) International Patent Classification (IPC):
*A61K 31/015* (2006.01)    *A61K 31/05* (2006.01)
*A61K 31/11* (2006.01)    *A61K 31/35* (2006.01)
*A61K 31/7048* (2006.01)    *A61L 9/00* (2006.01)
*A61M 15/00* (2006.01)    *A61P 31/00* (2006.01)
*A61P 31/04* (2006.01)    *A61P 31/12* (2006.01)
*A61P 31/14* (2006.01)    *A61P 31/16* (2006.01)
*A61P 31/20* (2006.01)    *F24F 8/15* (2021.01)

(52) Cooperative Patent Classification (CPC):
(C-Sets available)
A61K 31/35; A61K 31/015; A61K 31/05;
A61K 31/11; A61K 31/7048; A61K 31/732;
A61K 31/734; A61L 9/00; A61M 11/005;
A61M 15/02; A61M 15/08; A61P 31/00;
A61P 31/04; A61P 31/12; A61P 31/14;      (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **MVS Pharma GmbH**
**70771 Leinfelden-Echterdingen (DE)**

(72) Inventors:
• **PROKSCH, Rainer**
**70771 Leinfelden Echterdingen (DE)**
• **GEORGAKIS, Michail**
**54453 Thessaloniki (GR)**
• **KÜNNEMANN, Eva**
**8400 Winterthus (CH)**

(74) Representative: **Flügge, Katharina**
**Meissner Bolte Patentanwälte Rechtsanwälte**
**Partnerschaft mbB**
**Alter Wall 32**
**20457 Hamburg (DE)**

(54) **COMPOSITIONS AND METHODS FOR THE PROPHYLAXIS OF AN INFECTION**

(57) The present invention relates to compositions for use in the prophylaxis of an infection with infectious particles, including viruses, such as Sars-CoV-2, bacteria and fungi; to methods for the prevention of their transmission, including the use of compounds like geranial, eucalyptol, limonene, thymol, citral, carvacrol and hesperidin; and to a system containing a means for delivering at least one inhibiting substance against infectious particles.

Fig. 1

(52) Cooperative Patent Classification (CPC): (Cont.)
**A61P 31/16; A61P 31/20;** A61M 11/042;
A61M 2205/0205; A61M 2205/50;
A61M 2205/588; A61M 2205/84; A61M 2209/10;
A61M 2250/00; F24F 8/15

C-Sets
**A61K 31/11, A61K 2300/00;**
**A61K 31/7048, A61K 2300/00;**
**A61K 31/732, A61K 2300/00;**
**A61K 31/734, A61K 2300/00**

**Description**

**BACKGROUND**

The present invention relates to a composition for use in the prophylaxis of an infection with infectious particles, a method for the prophylaxis of the transmission of infectious germs, a method for blocking a receptor of an infectious germ and / or for binding to a binding site of an infectious germ, a delivery system containing a means for delivering at least one inhibiting substance against infectious particles and related uses.

Since a long time diseases exist in human communities that are transmitted via air because of small particles/particulates, solid or liquid matter suspended in the air, which can pass through the air over time and distance. Diseases capable of airborne transmission include human and veterinary maladies. The relevant pathogens can be viruses or bacteria, where in general the whole particles are transmitted, or fungi, where mostly spores are spread.

Some virus examples causing diseases are the Influenza virus A and B (causing influenza infection), the Respiratory Syncytial Virus (RSV; causing RSV infection), the SARS-CoV-2 virus (causing Coronavirus disease 2019), Mycobacterium tuberculosis (causing Tuberculosis), paramyxoviruses as the Measles virus (causing Measles) and Varicella zoster virus (causing Chickenpox and/or Herpes zoster) as well as adenovirus of subtype B.

Examples of diseases which are caused by bacteria are e.g. scarlet fever and meningococcal infections.

Some fungus examples causing diseases are Aspergillus sp. (causing Aspergillosis) and Mucorales sp. (causing Mucormycosis).

The infectious particles may be spread through breathing, exhaling, talking, coughing, sneezing, or any activities which generate aerosol particles or droplets. Aerosols are aqueous, solid, or oily suspended matter, or tiny particles that because of their low weight may float in the air for a long time before they sediment. Droplets with a diameter of more than 5 μm sink rapidly in the air and are therefore only transmitted up to around one meter. However, droplets of saliva can also stick to objects or surfaces. They can also reach the interior of the body through one's hand if - after having contact to an infected object or surface - the hand touches the mucous membrane of mouth, nose or eyes. Some pathogens can float in the air in droplets of very small size (<5 μm) for a long time and can thus be spread over great distances.

Coronavirus disease 2019 (COVID-19) is caused by severe acute respiratory syndrome coronavirus 2 (SARS-CoV-2). COVID-19 is a catastrophic disease that presently infects millions of people worldwide. To date, however, COVID-19 has spread in nearly all parts of the world and has even led to lockdowns in different areas. Since SARS-CoV-2 is propagated, people cannot meet and live as before. In several areas walking in town and shopping is only possible with a facial mask covering nose and mouth. Longer meetings with more people have been or are prohibited depending on the status of the pandemic situation. Concerts and other cultural events and activities where many people come together had been cancelled and new opportunities are and have to be developed to make events possible in secure surroundings. Always there is a fear of infection. In the past, everyone could see the face including the mouth of their conversation partner, but this is mostly no longer possible.

Since the COVID-19 epidemic people use facial masks and/or shields to protect themselves against transmission of the SARS-CoV-2 virus. These facial masks and/or shields cover mouth and nose. Some facial masks, like FFP2 masks, have specificities on particle-filtering respiratory protection, many others have not, they can even be self-made.

In the prior art, mouth sprays are disclosed that claim general unspecific antiviral activity. However, even when using these sprays, contact between people is still not possible, as they are not specifically developed for the purpose of protection.

Accordingly, there is a need for products and/or methods which allow the inhibition of the airborne transmission of infectious agents or particles. Thus, the problem underlying the present invention is to provide products and/or methods which allow the inhibition of the airborne transmission of infectious agents or particles. Another problem underlying the present invention is to provide products and/or methods which allow to overcome the pandemic situation in view of the SARS-CoV-2 virus and which allow individuals to return to a more "normal" life as before the pandemic situation.

**DESCRIPTION OF THE INVENTION**

This need is met by the present invention with compositions for use in the prophylaxis of an infection, methods for the prophylaxis of the transmission of infectious germs, a delivery system and related uses.

In a first aspect, the problem underlying the present invention is solved by a composition containing at least one substance with a binding energy between this substance and a binding site of an infectious particle, preferably a virus, bacterium or fungus, not exceeding -6.0 kcal/mol, preferably not exceeding -6.5 kcal/mol, more preferably not exceeding -7.9 kcal/mol for use in the prophylaxis of an infection with the infectious particle, preferably the virus, bacterium or fungus.

In a preferred embodiment, it is preferred that the prophylaxis comprises the prevention of the transmission of viruses, bacteria and / or fungi between two or more individuals or comprises the prevention of the transmission of viruses, bacteria and / or fungi via infected air or surface.

In another embodiment, it is preferred that the prophylaxis comprises the inhibition of the transmission of viruses, bacteria and / or fungi via infected air or surface.

In a preferred embodiment, the prevention of the transmission of viruses, bacteria and / or fungi between two or more individuals or the prevention of the transmission of viruses, bacteria and / or fungi via infected air or surface lasts for at least 0.5, 1, 2, 3, 4, 5 or more hours after the utilization of the composition according to the present invention.

Furthermore, it is preferred that the composition according to the present invention is utilized, preferably according to a method according to the present invention, every 0.5, 1, 2, 3, 4, 5 or more hours.

In another embodiment of the composition according to the present invention the composition is preferably an aqueous or non-aqueous solution or dispersion, or a phase stored in and delivered from a mineral, zeolite or another porous sorbent with or without a permeable membrane.

In a preferred embodiment of the composition according to the present invention the composition comprises at least one fragrance and / or flavoring substance in a concentration of at least 0.001 % by weight, preferably of at least 0.01 % by weight, more preferably of at least 0.1% by weight, in each case based on the total weight of the composition.

In a preferred embodiment of the composition according to the present invention the composition is preferably a spray, a lozenge, a pastille, a chewing gum or a lollipop.

In another preferred embodiment, of the composition according to the present invention, preferably according to at least one of the preceding embodiments the composition comprises at least one further excipient selected from the group comprising buffers, ethanol, film-forming agents, preservatives, stabilizers and matrix compounds.

In a preferred embodiment, of the composition according to the present invention the prophylaxis is the prophylaxis of an infection with the SARS-CoV-2 virus, preferably the prophylaxis of the transmission of SARS-CoV-2 viruses between individuals and/or the prophylaxis of an infection of one or more individuals via infected air and/or surface with the SARS-CoV-2 virus.

In a second aspect, the problem underlying the present invention is solved by a method for the prophylaxis of the transmission of infectious germs, preferably viruses, bacteria and / or fungi, between individuals comprising the step

(a) distributing a composition containing at least one substance with a binding energy between this substance and a binding site on an infectious particle, preferably a virus, bacterium or fungus, not exceeding -6.0 kcal/mol, preferably not exceeding -6.5 kcal/mol, more preferably not exceeding -7.9 kcal/mol, into an oral and / or nasal cavity of an individual or several individuals and / or into a preferably closed room or architectural space.

In a preferred embodiment, the prophylaxis of the transmission takes place by blocking a receptor of a virus, bacterium or fungal particle with the substance and / or by binding of the substance to a binding site of a virus, bacterium or fungus. If e.g. the virus-receptor interaction is blocked, the virus cannot enter into a cell to replicate, at least for a certain time period. As an example, on a SARS-CoV-2 virus there are about 100 spike glycoproteins that are binding to the receptor ACE2 (angiotensin converting enzyme) that is also expressed on the epithelial cells of the airways. By binding or docking to the spike glycoproteins those can not bind anymore to the ACE2 receptors on the epithelial cells of the airways. Infection and/or virus reproduction might be inhibited, accordingly. Additionally, it was measured that on average, there are $6.8 * 10^5$ viral RNA copies, and maximal $7.1 * 10^8$ viral RNA copies, what corresponds to the number of viruses per throat swab of infected, ill persons. The average viral load in sputum was measured to be $7.00 \times 10^6$ copies per ml, with a maximum of $2.35 \times 10^9$ copies per ml.

In a third aspect, the problem underlying the present invention is solved by a method for blocking a receptor of an infectious germ, preferably a virus, bacterium or fungus and / or for binding to a binding site of an infectious germ, preferably a virus, bacterium or fungus comprising the step

(a) distributing a composition containing at least one substance with a binding energy between this substance and a binding site on an infectious particle, preferably a virus, bacterium or fungus, not exceeding -6.0 kcal/mol, preferably not exceeding -6.5 kcal/mol, more preferably not exceeding -7.9 kcal/mol, into an oral and / or nasal cavity of an individual or several individuals and / or into a preferably closed room or architectural space.

If e.g. the virus-receptor interaction is blocked, the virus is inactive as it cannot enter into a cell to replicate, at least for a certain time period. If the binding site of an infectious germ is blocked or occupied by a substance or composition, the germ also becomes inactive, at least for a certain time period.

In a preferred embodiment, the composition or method, preferably according to at least one of the preceding embodiments, is provided wherein the at least one substance is selected from the group consisting of Hesperidin, Naringin, Caflanone, Glycoside diosmin, Hesperetin, Myricetin, Linebacker, Equivir, 2- hydroxy - propyl- β- cyclodextrin, 2-hydroxy- propyl —γ-cyclodextrin, Randomly methylated β- cyclodextrin, Sulfobutyl ether β - cyclodextrin, Caffeic acid, Propolis, Thymol, Limonene, Myrcene, Carvacrol, Geranial, Eugenol, β- caryophellene, eucalyptol, p- cymene, linalool, 2,5,5 - Trimethyl- 3,6-heptadien, 2- ol, Glycyrrhizin, Resveratrol, Reserpine, Aescin, Cepharanthine, Theavlavin, Myricetin, Scutellarein, Chalcones I-IX, Emodin, Celastrol, Apigenin, Tomentin A, Anethol, Geraniol, Pulegone, Lycorine, Homoharrintonine, Silvestrol, Ouabain, Tylophorine, Aloe vera oil Tea tree oil, Persica components, (E,E) - α - farnesene, (E,E) - farnesol, (E) - Nerolidol, α- bulnesene, Eremanthin, β- sesquiphellandrene, (Z) - spiroether, (E) - cinnamyl acetate, (E) - β - farnecene, Geramyl formate, Cinnamaldehyde, Menthol, Carvacrol, Tomentin B, Hesperetin, Saikosaponin B2, Jubanine H, Iguesterin, APA, Belutonic Acid, Betulinic Acid, Curcumin, Indigo, Psoralidin, Tannic acid, Silvestrol, Gllycyrrhizin, Saikosaponin A, Tetra-O-galloyl-β-D-glucose, Luteolin, Sinigrin, β-sitosterol, amentoflavone, quercetin, isobavachalcone, tometin A, tometin B, tometin E, 3'-O-methyldiplacol, isoliquiritigenin, quercetin, kaempferol, kazinol F, broussochacone B, papyriflavonol A, terrestrimine, blancoxanthone, pyranojacareubin, tylophorine, 7-methosycryptopeurine, jubanine G, jubanine H, nummularine B, tingenone, iguesterin, pristimererin, dihydrotanshinone I, cryptotanshinone, tanshinone IIA, schimperinone, xanthoangelol, hirsutenone, rubranoside, curcumin, Santolina triene, α-pinene, camphene, viridiflorol, terpin-4-ol, 1,8-cineole, methylchavicol, camphor, myrcene, 1,8-cineole, β-caryophellene, linalool, gerenyl acetate, β-himachalene, α-humulene, γ-himachalene, γ-terpin-7-al, γ-terpinene, β-pinene, cuminaldehyde, δ-cadinene, germacrene D, α-copaene, germacrene B, β-bisabotene, limonene, n-menthal, 8-dien-10-al, eugenol, carvacrol, cinnamaldehyde, viridiflorol, p-cymene, (Z)-caryophyllene, methylchavicol, trans-anthole, α-terpinyl acetate, β-phellandrene, Trans nerolidol, cis-dihydrocarvacol, eucalyptol, thymol, longifolene, thymoquinone, thymohydroquinone, α-thujene, p-cymene, borneol, camphene, camphor, gereniol, bornyl acetate, α-terpinolene, benzoylacetate, α-thujone, eugenylacetate, terpineol, β-thujone, terpine-4-ol, trans-nerolidol, β-eudesmol, Terpineol, α-terpineol, m-cymene, myrtenol, camphor, 1,8-cineole, carvacrol, α-bisabolol, hexadecanoic acid, α-pinene, β-pinene, β-phellandrene, δ-elemene, farnesene, α-curcumene, selina-4,7(11)-diene, β-bisabolene, piperitone, piperitenone, dillapiole, γ-terpinene, sabinene, thymol-methylether, sardinia:β-bisbolene, myacene, limonene, elemicin, aneocallitropsene, isocaryophyllen-14-al, 14-hydroxy-β-caryophyllen, caryophyllene oxide, E-β-caryophyllene, thymol, p-cymene, γ-terpinene, terpinen-4-ol, linalool, geraniol, trans-anethole, anethol, fenchone, sabinone, terpinolene, safrote, trans-cinnamaldehyde, eugenol isomer, camphor and nigellone. Preferably, the at least one substance is selected from the group consisting of geranial, eucalyptol, limonene, thymol, citral, carvacrol and Hesperidin.

If the at least one substance according to the composition or the method of the present invention is an essential oil, which could be hydrophobic, it is sometimes preferred that e.g. a carrier for the essential oil is used so that the interactions between the hydrophobic essential oil and the germ particles which are carried in water droplets (as e.g. the SARS-CoV-2 virus particles) would not be prohibited (or at least become less possible) in air. Another possibility would be to disperse the essential oil in water droplets, requiring a carrier, a surfactant or a supersonication.

In a preferred embodiment, the method according to at least one of the preceding embodiments is provided wherein step (a) is

(a) distributing a composition containing at least one substance with a binding energy between this substance and a binding site on an infectious particle, preferably a virus, bacterium or fungus, not exceeding -6.0 kcal/mol, preferably not exceeding -6.5 kcal/mol, more preferably not exceeding -7.9 kcal/mol, into an oral and / or nasal cavity of an individual or several individuals shortly before entering a preferably closed room or architectural space.

In a preferred embodiment, the method according to at least one of the preceding embodiments is provided wherein before step (a) takes place, step (a-1) is provided, wherein step (a-1) is

(a-1) distributing a composition containing at least one substance with a binding energy between this substance and a binding site on an infectious particle, preferably a virus, bacterium or fungus, not exceeding -6.0 kcal/mol, preferably not exceeding -6.5 kcal/mol, more preferably not exceeding -7.9 kcal/mol, into the preferably closed room or architectural space.

In a preferred embodiment, the method according to at least one of the preceding embodiments is provided wherein additional step (b) takes place after step (a), wherein step (b) is

(b) distributing a composition containing at least one substance with a binding energy between this substance and a binding site on an infectious particle, preferably a virus, bacterium or fungus, not exceeding -6.0 kcal/mol, preferably not exceeding -6.5 kcal/mol, more preferably not exceeding -7.9 kcal/mol, into the preferably closed room or architectural space as long as the individual or the individuals are present.

In a preferred embodiment, the method according to at least one of the preceding embodiments, is provided wherein the distribution step is provided by spraying or smoking the composition, preferably with an amount of 0.01 to 10 μg, more preferably with an amount of 0.1 to 1.0 μg, of substance per spray burst or inhalation. It is further preferred that the amount of one spray burst is a drop of about 50-100 μg. Preferably, the concentration of the at least one substance ranges between 0.5 and 20 mM.

In a preferred embodiment, the method according to at least one of the preceding embodiments, is provided wherein the distribution in step a) takes place into the oral and / or nasal cavity, preferably of two or more individuals who are in spatial proximity to one another. However, individuals can get infected also without being in spatial proximity if viruses, bacteria and / or fungi are able to stay airborne (suspended) in closed spaces, as e.g. the SARS-CoV-2 virus. Thus, it is possible to become infected even without being in close proximity with another person. However, the prophylaxis of the transmission of infectious germs according to the present invention would work in this case as well. The method itself acts like a mask when it remains in the nasal and mouth cavities and covers also the mucous membranes.

In a preferred embodiment, the method according to at least one of the preceding embodiments, is provided wherein the one individual or at least one of the several individuals has already been vaccinated against the infectious germ, preferably the virus, the bacterium or the fungus.

In a preferred embodiment, the method according to at least one of the preceding embodiments, is provided wherein the infectious germ is a SARS-CoV-2 virus, an Influenza virus A or B, a Respiratory Syncytial Virus (RSV), a paramyxoviruses, preferably a Measles virus, a Varicella zoster virus, an adenovirus of subtype B or a Mycobacterium tuberculosis or a bacterium causing scarlet fever or meningococcal infections, or an Aspergillus or Mucorales fungus, preferably a SARS-CoV-2 virus.

In a preferred embodiment, the method according to at least one of the preceding embodiments, is provided wherein the individual or the individuals are, independently of one another, a human or an animal or a plurality of humans or a plurality of animals, preferably one or more humans.

In a preferred embodiment, the method according to at least one of the preceding embodiments, is provided wherein the architectural space is an office or an office building, a school room, a hotel, an exhibition hall, a sports hall, an opera, a large event room, a shopping center or a concert hall, or a mode of transport, in particular a bus, a train, a taxi, a ship, an aircraft or a plane.

In a preferred embodiment, the method according to at least one of the preceding embodiments, is provided, wherein the composition is a composition according to the present invention, preferably according to at least one of the preceding embodiments.

In a preferred embodiment, the method according to at least one of the preceding embodiments, is provided, wherein step (a) is executed every 0.5, 1, 2, 3, 4, 5 or more hours.

In a preferred embodiment, the method according to at least one of the preceding embodiments, is provided, wherein the prophylaxis of the transmission of infectious germs, the blocking of a receptor of an infectious germ or the binding to a binding site of an infectious germ, lasts for at least 0.5, 1, 2, 3, 4, 5 or more hours after executing step (a).

In a preferred embodiment, the method according to at least one of the preceding embodiments, is provided, wherein in an additional step a second binding or a crosslinking can be achieved. Infectious germs, as e.g. priones, which consist mainly only of protein, could be inhibited by a complete masking and crosslinking by and of one or more of the substances according to the present invention on the surface of the infectious germs.

In a fourth aspect, the problem underlying the present invention is solved by a use of a composition or a method according to the present invention for the prophylaxis of an infection with an infectious particle, preferably a virus, bacterium or fungus and / or for the protection of the transmission of an infectious particle, preferably a virus, bacterium or fungus, from an infected individual to a non-infected or to an infected individual.

In a preferred embodiment, the use according to the present invention, is provided wherein the prophylaxis comprises the prevention of the transmission of an infectious particle, preferably a virus, bacterium or fungus, between two or more individuals or comprises the prevention of the transmission of an infectious particle, preferably a virus, bacterium or fungus, through infected air or surface.

In a preferred embodiment, the use according to the present invention, preferably according to at least one of the preceding embodiments, is provided wherein the prophylaxis is the prophylaxis of an infection with a SARS-CoV-2 virus.

In a fifth aspect, the problem underlying the present invention is solved by a use of a composition according to the present invention for recognition, preferably in the form of a recognition signal, alert or indicator. In a preferred use of a composition according to the present invention the composition has a specific smell. Through this specific smell of the composition according to the present invention people when recognizing this specific smell can be sure that their direct surrounding is safe in view of an infection with a specific virus, bacterium or fungus, most preferably with the SARS-CoV-2 virus. The smell is preferably based on the smell of citral, which combines the effects of inactivating an infectious particle, preferably a virus, and providing - by its smell - a form of a recognition signal, alert or indicator. In a preferred embodiment of the use according to the present invention a specific smell provides the recognition signal, alert or indicator to individuals that they and/or their environment is or are safe in view of the SARS-CoV-2 virus. This use according to the present invention is preferably used when individuals are in closed rooms or in architectural space as an office or an office building, a school room, a hotel, an exhibition hall, a sports hall, an opera, a large event room, a shopping center or a concert hall, or a mode of transport, in particular a bus, a train, a taxi, a ship, an aircraft or a plane.

Thus, in a preferred embodiment, the use of a composition according to the present invention for recognition is wherein the recognition is a signal of security and / or protection. In other words: when a composition according to the present invention, preferably having a specific smell, is used in a closed room or in an architectural space as an office or an office building, a school room, a hotel, an exhibition hall, a sports hall, an opera, a large event room, a shopping center or a concert hall, or a mode of transport, in particular a bus, a train, a taxi, a ship, an aircraft or a plane, individuals recognizing this specific smell obtain the information, i.e. the signal, through this specific smell that this area is safe in view of the transmission of an infectious particle, preferably a virus, bacterium or fungus, most preferably of the SARS-CoV-2 virus.

In a sixth aspect, the problem underlying the present invention is solved by a use of a delivery system for the composition according to the invention, preferably according to at least one of the preferred embodiments, in an air purification system and/or air conditioning system wherein the air purification system and/or the air conditioning system comprises at least one solid carrier, preferably a porous solid, a mineral, or a porous polymeric material, that comprises, preferably carries, the composition.

In a preferred embodiment, the use of such a delivery system according to the present invention, is provided, wherein the system further comprises a chemically gated system, preferably a ligand on a substrate, a permeable membrane, or a porous polymeric material.

In a preferred embodiment, the use of such a delivery system according to the present invention, is provided, wherein the system further comprises a programmable electronic dispenser.

In a seventh aspect, the problem underlying the present invention is solved by a delivery system containing a means for delivering at least one inhibiting substance against infectious particles via air into an empty room, a mouth and/or nose spray containing at least one inhibiting substance against these infectious particles for use by an individual when entering a or the room and/or a means for delivering at least one inhibiting substance against the infectious particles via air into a or the room, if one, two or more individuals are present. Preferably, the means for delivering at least one inhibiting substance against infectious particles in such an inventive delivery system is or contains a composition according to the present invention.

In a preferred embodiment, the delivery system according to the present invention, is an air purification system and/or air conditioning system.

In a preferred embodiment, the delivery system according to the present invention, further comprises at least one solid carrier, preferably a porous solid, a mineral, or a porous polymeric material, that preferably comprises the means for delivering at least one inhibiting substance against infectious particles, preferably a composition according to the present invention.

In a preferred embodiment, the delivery system according to the present invention further comprises a chemically gated system, preferably a ligand on a substrate, a permeable membrane, or a porous polymeric material.

In a preferred embodiment, the delivery system according to the present invention further comprises a programmable electronic dispenser.

Preferably, the delivery system according to the present invention contains a means for delivering at least one inhibiting substance against infectious particles via air into an empty room, wherein the at least one inhibiting substance against infectious particles is delivered via air into an empty room and creates a distribution of the at least one inhibiting substance in a sufficient concentration in the indoor air to obtain a "cleaned air". The distribution profile throughout a room depends heavily on the velocity of air coming out of the delivery system, e.g. the air conditioner, the mixing of the delivered air including the at least one inhibiting substance with the, preferably "infected", air, i.e. air including infectious particles, and - highly important - the relative weights. Exhalation by an individual through normal breathing generally has lower velocities (this might be higher during coughing and sneezing or even working out intensely) and higher molecular weights (of the virus) to deal with, thus, if direct transmission of the infectious particles, as the SARS-CoV-2 virus, should be prohibited by a mouth and/or nose spray containing at least one inhibiting substance, it has to be considered that by exhalation the infectious particles, as the SARS-CoV-2 virus, merely are emitted around the individuals and do not extensively distribute in the air (especially, the SARS-CoV-2 virus itself is too heavy to be dispersed and distributed over long distances. However, if people come into the area/a surrounding where an infected individual has exhaled, they can become infected. Furthermore, it has to be considered that while the disclosed behaviour is (mostly) true for non-moving air, most air is moving due to thermal gradients, and people moving in a space, thus there is always a spread of infectious particles, if present. In addition to that, the diffusion according to fick's law even under zero relative velocities has to be considered.

In a preferred embodiment, the delivery system according to the present invention allows efficient contact times and areas between the at least one inhibiting substance against infectious particles, which is preferably hydrophobic, and water droplets so that the at least one inhibiting substance is equally mixed or homogenized into/with the water droplets when distributed into the air. Preferably, the delivery system according to the present invention includes an ultrasonication mixer. This ultrasonication mixer can produce stable micro-dispersions of the at least one inhibiting substance against infectious particles, preferably of essential oils, in dispensers, which can be used as sprays from air conditioning units. An advantage is that the micro-dispersions produced by the ultrasonication mixer stay similar for a long time, preferably for at least a month.

According to the present invention, instead of an ultrasonication mixer a high shear mixer can be used which provides the same advantages as an ultrasonication mixer.

In a preferred embodiment, the delivery system according to the present invention includes an electrosprayer that can produce an electro dynamic phenomenon (Taylor cone) directly from a container via the application of a suitable electrical current and voltage, and flow through an appropriate nozzle. The electrosprayer is extremely efficient in surface desinfection. In the case of the electrosprayer, the device is installed in the delivery system according to the present invention, preferably the air conditioning unit. This is in contrast to the embodiments including an ultrasonication mixer or a high shear mixer where only the stable dispersion has to be placed in the air conditioning unit. The electrodynamic effect will preferably create mono-dispersions. This can preferably allow an optimization of the efficiency against the infectious particles, preferably the SARS-CoV-2 virus in the water droplets in the air.

In a preferred embodiment, the delivery system according to the present invention allows a mixing process based on the use of small size, low molecular weight surfactants. A mixture process with such surfactants will produce stable micro-dispersions of the at least one inhibiting substance against infectious particles, preferably an essential oil, in dispensers. Such dispersions could be used as sprays from air conditioning units and stay similar for a long time, preferably for at least a month. The appropriate surfactants will also be non-toxic, non-irritating and considered safe for use in food and air purification applications.

In a preferred embodiment, the delivery system according to the present invention allows a mixing process based on the use of known safe polymeric carriers, e.g. with derivatives of β-cyclodextrin. A mixture process with such polymeric carriers will produce stable micro-dispersions of the at least one inhibiting substance against infectious particles, preferably an essential oil, in dispensers. Such dispersions could also be used as sprays from air conditioning units and stay similar for a long time, preferably for at least a month.

To maximize the contact time and areas of the dispersed at least one inhibiting substance against infectious particles, preferably an essential oil, to the water droplets a combination of at least two of the above mixing processes and/or mixing devices is preferred in a delivery system according to the present invention.

In a preferred embodiment, the delivery system according to the present invention is used with at least one hydrophilic inhibiting substance against infectious particles, preferably an essential oil, and/or other natural compounds disclosed herein. For such embodiment, a use of any of the dispersion and/or mixing techniques mentioned above is not obligatory, however, it is suggested to improve the contact efficiency of the given system. It is suggested, that using any of the above-mentioned techniques will also create a longer residence time of the at least one hydrophilic inhibiting substance against infectious particles, preferably an essential oil, in air due to two factors a) smaller sizes and reduction of aggregation, that will allow the lighter particles to stay airborne for longer times and thus it will provide a higher chance for interacting with an infectious particle, preferably a virus particle, and b) stabilization forces among the at least one hydrophilic inhibiting substance against infectious particles, preferably essential oil particles, due to supersonication, surfactants, carriers, etc. will allow for a lighter distribution in the indoor environment. For most cases the supersonication is preferred, and it consumes less energy.

According to the present invention, the at least one substance in a composition according to the present invention or in a method according to the present invention, preferably hydrophobic or hydrophilic, preferably inhibiting, can be an antibody, a fragment of an antibody, a nanobody, an artificial molecule or the like.

The term "via infected air or surface" as used herein refers to that infectious germs and/or particles can be transmitted from one individual to another via infected air, e.g. air having infectious germs and/or particles airborne (suspended) in the air or via infected surfaces, e.g. by finger contact on a surface whereon infectious germs and/or particles are attached.

The term "germ" as used herein refers to a usually small organism as e.g. a virus, a bacterium or a fungus that can cause a disease.

The term "infectious particles" as used herein refers to particles of viruses, bacteria or fungi or viruses, bacteria or fungi, in each case as a whole, which can cause diseases or illnesses in individuals, preferably humans.

The present invention is further illustrated by the following figures and examples from which further features, embodiments, aspects and advantages of the present invention may be taken.


**BRIEF DESCRIPTION OF THE FIGURES**

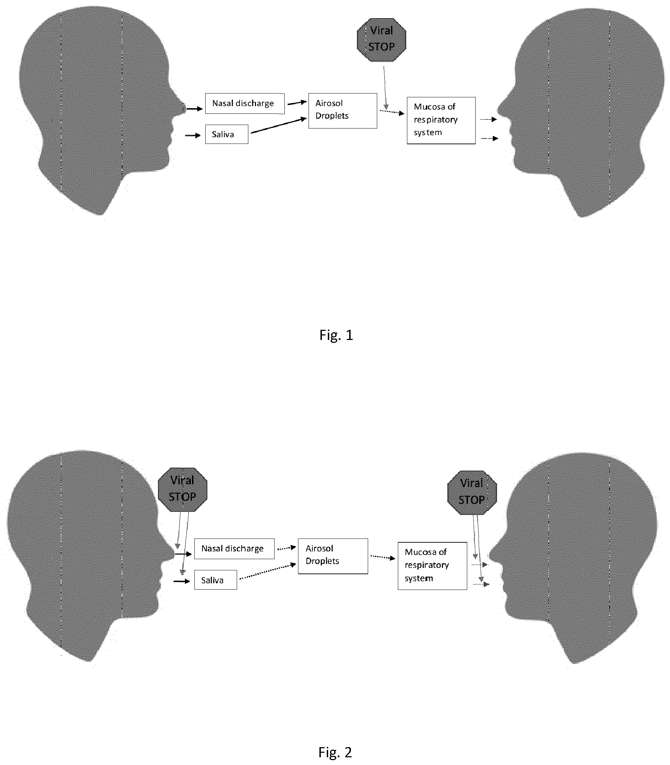

**Fig.** 1 shows a scheme for a method for the prophylaxis of the transmission of infectious germs via air according to the present invention.

In Fig. 1 transmission of infectious germs via air is inhibited by using a room/air spray according to the present invention when being in spatial proximity to one another.

**Fig.** 2 shows another scheme for a method for the prophylaxis of the transmission of infectious germs via air according to the present invention.

In Fig. 2 transmission of infectious germs via air is inhibited by using a mouth and/or nose spray according to the present invention when being in spatial proximity to one another.

## EXAMPLES

### Example 1 — Docking approach

A molecular docking approach was employed to calculate the binding energies of a group of essential oils on surface proteins of SARS-CoV-2 that utilizes exact, atomistic scale models of the ligands (essential oils) and the target molecules (SARS-CoV-2 proteins). It calculates their interactions based on hydrogen bonding, dispersion forces, London forces, hydrophobic interactions, and electrostatic interactions.

The first step was to create the essential oils models. Those models were created in HyperChem v8, HyperCube Inc., USA. All models were generated and optimized under Density Functional Theory, mainly to obtain the exact structures, including their electronic charge distribution. Twelve essential oils were created via this approach, with a target to continuously enrich this collection. The model creation process included drawing, initial modelling, a geometry optimization and a molecular dynamics step. Geometry Optimizations lead to energetic minima, which in most cases are 'local' ones, whereas the 'global' minima are of the highest importance. For that reason, a Molecular Dynamics step was employed after each Geometry Optimization, to 'break free' of the energy trap and continue the search for a 'deeper' energetic minimum. This methodology was repeated up to twenty times for each essential oil molecular model generated.

The second step was the preparation of the SARS-CoV-2 proteins that are the binding targets of the essential oil molecules. Four proteins, namely (PDB ID): RdRp (6M71), Spike ectodomain (6VYB), PIPr (6W9C) and MPro (6Y2E) were employed. The crystallographic structures are offered free of charge in a number of databases, and the Protein Data Bank (RCSB.ORG) was used as source. A series of preparation modification were carried out in Chimera environment (CSF Chimera, developed by the Resource for Biocomputing, Visualization, and Informatics at the University of California, San Francisco, with support from NIH P41-GM103311) under the AMBER FF14SB force field:

- Chain A of the RdRp protein was used (6M71_A)
- N-acetyl glucosamine was removed from the spike protein (6VYB)
- Chain A of MPro was used (6Y2E_A)
- Missing protons were added where required
- Crystallographic water molecules were removed where required

The generated models were in mol2 format and were fed to AutoDockTools software (v4.2, The Scripps Research Institute, USA), which in turn generated pdbqt files for AutoDock Vina (O. Trott, A. J. Olson, AutoDock Vina: improving the speed and accuracy of docking with a new scoring function, efficient optimization and multithreading, Journal of Computational Chemistry 31 (2010) 455-461). The pdbqt format preserves the charges from the mol2 files.

The third step was to use AutoDock Vina to execute the molecular docking simulations of the 12 ligands vs the 4 proteins. The Lamarckian genetic algorithm 24 was adopted for the process, using 10-40 evaluations for run and 25 million iterations producing the results. The iterative local search method of AutoDock Vina was used for calculating the interaction potentials in the system. An initial low spacing was used, which was substituted with a much finer one after the initiation of the process, to produce higher accuracy results. Analysis of all hits and their binding energies was the last step of the process.

The essential oils that were studied, accompanied by the best binding energies to the four proteins are presented in **Table 1**. The multiplicity exhibits the number of binding pockets in the total of the four proteins.

**Table 1**: Molecular Docking results for the 12 essential oils vs RdRp, MPro, Spike and PIPr of SARS-CoV-2

| Compound | Binding energy [kcal/mol] | Multiplicity [# of sites in total] |
|---|---|---|
| Geranial | -7.7 | 6 |
| Eucalyptol | -8.3 | 6 |
| Caffeic acid | -6.1 | 3 |
| Myrcene | -6.0 | 3 |
| Limonene | -7.4 | 4 |

(continued)

| Compound | Binding energy [kcal/mol] | Multiplicity [# of sites in total] |
|---|---|---|
| Thymol | -7.3 | 4 |
| Hesperidin | -8.5 | 6 |
| Carvacrol | -5.7 | 2 |
| Linalool | -5.5 | 2 |
| B-caryophyllene | -6.1 | 3 |
| Hesperitin | -5.4 | 2 |
| Curcumin | -6.9 | 2 |

## Example 2 - Calculation of Dissociation constant

Calculation of dissociation constants from binding energies

For the binding of receptor and ligand molecules in solution, the molar Gibbs free energy, or the binding affinity is related to the dissociation constant via

$$\Delta G = RT \ln \frac{K_d}{c^{\ominus}},$$

$\Delta G$: molar Gibbs free energy
$R$: ideal gas constant, R= 8.314 J K1 mol$^{-1}$ (=1.9872 * 10$^{-3}$ kcal K$^{-1}$ mol$^{-1}$ )
$T$: temperature in K, for example at 20°C: T = 273 K + 20 K = 293 K
$K_d$: dissociation constant
$c^{\ominus}$: standard reference concentration, $c^{\ominus}$ = 1 mol/L

Reforming the term to calculate $K_d$ from $\Delta G$ yields the following equation:

$$K_d = e^{\Delta G/RT} * c^{\ominus}$$

For example, if the binding energy is -7.7 kcal/mol, then:

$$K_d = e^{\frac{-7.7 \text{ kcal*mol}^{-1}}{1.9872 * 10^{-3} \text{ kcal K}^{-1} \text{ mol}^{-1} * (293 \text{ K})}} * 1 \text{ mol/L} = 1.8 * 10^{-6} \text{ M}$$

The smaller the dissociation constant $K_d$, the higher the affinity.

The following equation allows the calculation of the percentage of occupancy of a protein with its ligand as a function of $K_d$ and the total concentrations of Protein P and Ligand L in solution from the chemical binding reaction:

$$P + L \underset{k_{off}}{\overset{k_{on}}{\rightleftharpoons}} PL$$

$$\frac{[P] \cdot [L]}{[PL]} = \frac{k_{off}}{k_{on}} = K_d$$

If there is much more ligand than protein, the concentration of free ligand stays approximately constant during the reaction. Therefore, if in this case $K_d$ and the starting concentration of ligand [L] are known, one can calculate the percentage of protein that has a ligand bound.

For example: If the starting concentration of the ligand is $2.2*10^{-4}$ M = 0.22 mM and as in the above example $K_d = 2.2 * 10^{-7}$ M this yields the information that the starting concentration is $1000 \times K_d$ and that consequently 99.9% of proteins have a ligand bound.

From a known $K_d$ also a starting concentration can be chosen in this way, that it is for example $1000 \times K_d$ and thereby it can be achieved that as a result 99.9% of proteins can be blocked with a ligand. Or if the substrate concentration is $100 \times Kd$ 99% of proteins will be blocked.

**Table 2**: Dissociation constant for 5 compounds

| Compound | Dissociation constant [M] | Concentration $1000 \times K_{diss}$ [M] | Solubility in water [M] |
|---|---|---|---|
| Geranial | $1.8*10^{-6}$ | $1.8*10^{-3}$ | $2.8*10^{-3}$ |
| Eucalyptol | $6.4*10^{-7}$ | $6.4*10^{-4}$ | $21*10^{-3}$ |
| Limonene | $3.0*10^{-6}$ | $3.0*10^{-3}$ | $1*10^{-4}$ |
| Thymol | $3.6*10^{-6}$ | $3.6*10^{-3}$ | $6*10^{-3}$ |
| Hesperidin | $4.5*10^{-7}$ | $4.5*10^{-4}$ | $8.3*10^{-3}$ |

In Table 2 the concentration to block 99.9% of proteins with a ligand were calculated. By comparing this concentration to the solubility of the substances in water it can be concluded that even higher concentrations than $1000 \times K_d$ can be used in aqueous spray solution. The substances are not toxic in these concentrations.

However, it has additionally to be considered, that when the solution is sprayed into the nose and/or the oral cavity it will be perhaps be diluted with saliva. Even if this dilution is 10 times (for example 100 μl solution is sprayed into a mouth with 1 ml saliva), the concentration of the substance will be $> 100 \times K_d$ and the amount of blocked proteins would be >99%.

The following calculation shows that the concentration of the substances is much higher than the protein concentration on the viruses, where there is a maximum of $2.35 \times 10^9$ copies per ml. On each virus there are about 100 spike proteins, therefore there would be a concentration of about $2.35 \times 10^{11}$ spike proteins per ml.

The concentrations of binding substances would be around $1*10^{-3}$ M.
$1*10^{-3}$ M = $1*10^{-3}$ mol/l = $1*10^{-3}$ $(6.022*10^{23})$ molecules / l = $6.022*10^{20}$ molecules / l = $6.022*10^{17}$ molecules / ml.

Therefore, there will be around one hundred million times ($10^8$ x) the number of substance molecules compared to spike protein molecules and at least one million times ($10^6$ x) the number of substance molecules compared to viruses.

## Example 3 - Compositions

Three compositions according to the present invention were prepared.

**Composition 1**

| |
|---|
| 5 mM Hesperidin |
| 0.5 mM Limonene |
| 2 mM Citral |
| 12 % Glycerol |
| 0.5 % Ethanol |
| 27 mM citric acid |
| 23 mM sodium citrate |

For 1 L 6.76 g Sodium Citrate dihydrate and 5.19 g citric acid were weighted and added to a 1 L beaker and filled with about 800 ml of sterile $H_2O$. This mixture was stirred until the salts were dissolved. As a next step, 342 μl Citral was added. 3.05 mg Hesperidin was weighted, diluted in 120 ml Glycerol and added to the mixture. Additionally, 81 μl limonene was mixed with 5 ml Ethanol and added to the rest. After addition of all these components, the formulation was stirred, and the pH was adjusted to 4.5. Finally, the volume was filled with sterile $H_2O$ to 1 L, and the formulation was sterile filtered before it was aliquoted into sterile spray bottles.

**Composition 2**

| |
|---|
| 20 mM Eucalyptol |
| 5 mM Thymol |
| 12 % Glycerol |
| 10 mM Benzoic acid |
| 13.5 mM Sodium benzoate |
| 0.1 % Ethanol |

For 1 L 1.22 g Benzoic acid and 1.95 g Sodium benzoate were weighted and added to a 1 L beaker and filled with about 800 ml of sterile $H_2O$. This mixture was stirred until the salts were dissolved. 782.3 µl Thymol and 3.31 µl Eucalyptol were added to 120 ml Glycerol and this mixture was added to the mixture with benzoic acid and benzoate. In the next step, 1 ml Ethanol was added to the rest and the formulation was stirred. The pH was controlled and adjusted to 4.5. Finally, the volume was filled with sterile $H_2O$ to 1 L, and the formulation was sterile filtered before it was aliquoted into sterile spray bottles.

**Composition 3**

| |
|---|
| 8 mM Hesperidin |
| 12 % Glycerol |
| 18.7 mM $Na_2HPO_4$ |
| 10.7 mM Citric acid |
| 1 % Ethanol |

For 1 L 3.33g $Na_2HPO_4$ and 2.06 g Citric acid were weighted, added to a 1 L beaker and filled with about 800 ml of sterile $H_2O$. This mixture was stirred until the salts were dissolved. 4.88 mg Hesperidin was diluted in 120 ml Glycerol and added to the mixture with $Na_2HPO_4$ and Citric acid. 10 ml Ethanol was added and mixed. The pH was controlled and adjusted to 4.5. Finally, the volume was filled with sterile $H_2O$ to 1 L, and the formulation was sterile filtered before it was aliquoted into sterile spray bottles.

**Claims**

1. A composition containing at least one substance with a binding energy between this substance and a binding site of an infectious particle, preferably a virus, bacterium or fungus, not exceeding -6.0 kcal/mol, preferably not exceeding -6.5 kcal/mol, more preferably not exceeding -7.9 kcal/mol for use in the prophylaxis of an infection with the infectious particle, preferably the virus, bacterium or fungus.

2. Composition according to claim 1 wherein the prophylaxis comprises the prevention of the transmission of viruses, bacteria and / or fungi between two or more individuals or comprises the prevention of the transmission of viruses, bacteria and / or fungi via infected air or surface.

3. Composition according to at least one of the preceding claims, wherein the composition comprises at least one further excipient selected from the group comprising buffers, ethanol, film-forming agents, preservatives, stabilizers and matrix compounds.

4. Composition according to at least one of the preceding claims, wherein the prophylaxis is the prophylaxis of an infection with the SARS-CoV-2 virus, preferably the prophylaxis of the transmission of SARS-CoV-2 viruses between individuals and/or the prophylaxis of an infection of one or more individuals via infected air and/or surface, preferably with the SARS-CoV-2 virus.

5. A method for the prophylaxis of the transmission of infectious germs, preferably viruses, bacteria and / or fungi, between individuals comprising the step

(a) distributing a composition containing at least one substance with a binding energy between this substance and a binding site on an infectious particle, preferably a virus, bacterium or fungus, not exceeding -6.0 kcal/mol, preferably not exceeding -6.5 kcal/mol, more preferably not exceeding -7.9 kcal/mol, into an oral and / or nasal cavity of an individual or several individuals and / or into a preferably closed room or architectural space.

6. A method for blocking a receptor of an infectious germ, preferably a virus, bacterium or fungus and / or for binding to a binding site of an infectious germ, preferably a virus, bacterium or fungus comprising the step

(a) distributing a composition containing at least one substance with a binding energy between this substance and a binding site on an infectious particle, preferably a virus, bacterium or fungus, not exceeding -6.0 kcal/mol, preferably not exceeding -6.5 kcal/mol, more preferably not exceeding -7.9 kcal/mol, into an oral and / or nasal cavity of an individual or several individuals and / or into a preferably closed room or architectural space.

7. Composition or method according to at least one of the preceding claims, wherein the at least one substance is selected from the group consisting of geranial, eucalyptol, limonene, thymol, citral, carvacrol and Hesperidin.

8. The method according to at least one of the preceding claims, wherein the distribution step is provided by spraying or smoking the composition, preferably with an amount of 0.01 to 10 μg, more preferably with an amount of 0.1 to 1.0 μg, of substance per spray burst or inhalation.

9. The method according to at least one of the preceding claims, wherein the infectious germ is a SARS-CoV-2 virus, an Influenza virus A or B, a Respiratory Syncytial Virus (RSV), a paramyxoviruses, preferably a Measles virus, a Varicella zoster virus, an adenovirus of subtype B or a Mycobacterium tuberculosis or a bacterium causing scarlet fever or meningococcal infections, or an Aspergillus or Mucorales fungus.

10. The method according to at least one of the preceding claims, wherein the architectural space is an office or an office building, a school room, a hotel, an exhibition hall, a sports hall, an opera, a large event room, a shopping center or a concert hall, or a mode of transport, in particular a bus, a train, a taxi, a ship, an aircraft or a plane.

11. The method according to at least one of the preceding claims, wherein the composition is a composition according to at least one of claims 1 to 4 and 7.

12. Use of the composition or the method according to at least one of the preceding claims for the prophylaxis of an infection with an infectious particle, preferably a virus, bacterium or fungus and / or for the protection of the transmission of an infectious particle, preferably a virus, bacterium or fungus, from an infected individual to a non-infected or to an infected individual.

13. Use according to claim 12, wherein the prophylaxis is the prophylaxis of an infection with a SARS-CoV-2 virus.

14. Use of a delivery system for the composition according to at least one of the claims 1 to 4 and 7 in an air purification system and/or air conditioning system wherein the air purification system and/or the air conditioning system comprises at least one solid carrier, preferably a porous solid, a mineral, or a porous polymeric material, that comprises the composition and wherein the system optionally comprises a chemically gated system, preferably a ligand on a substrate, a permeable membrane, or a porous polymeric material and/or optionally comprises a programmable electronic dispenser.

15. A delivery system containing a means for delivering at least one inhibiting substance against infectious particles via air into an empty room, a mouth and/or nose spray containing at least one inhibiting substance against these infectious particles for use by an individual when entering a or the room and/or a means for delivering at least one inhibiting substance against the infectious particles via air into a or the room if one, two or more individuals are present.

Fig. 1

Fig. 2

Europäisches
Patentamt

European
Patent Office

Office européen
des brevets

# EUROPEAN SEARCH REPORT

Application Number

EP 21 20 0432

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/191886 A1 (EYBNA TECH LTD [IL]) 30 September 2021 (2021-09-30) <br> * claims 1-30 * <br> * paragraph [0001] * <br> * paragraph [0015] * <br> * paragraph [0022] - paragraph [0043] * <br> * paragraph [0056] * <br> * paragraph [0066] - paragraph [0094] * <br> * paragraph [0095] - paragraph [0098] * <br> * paragraph [0222] - paragraph [0250]; examples 1-4 * <br> * paragraph [0251] - paragraph [0260]; example 5 * <br> * figures 1-22 * <br> * abstract * <br> * the whole document * <br> ----- | 1-15 | INV. <br> A61K31/015 <br> A61K31/05 <br> A61K31/11 <br> A61K31/35 <br> A61K31/7048 <br> A61L9/00 <br> A61M15/00 <br> A61P31/00 <br> A61P31/04 <br> A61P31/12 <br> A61P31/14 <br> A61P31/16 <br> A61P31/20 <br> F24F8/15 |

TECHNICAL FIELDS
SEARCHED      (IPC)

A61K
F24F
A61M
A61L
A61P

The present search report has been drawn up for all claims

| Place of search | Date of completion of the search | Examiner |
|---|---|---|
| The Hague | 17 February 2022 | Langer, Oliver |

EPO FORM 1503 03.82 (P04C01)

**ANNEX TO THE EUROPEAN SEARCH REPORT
ON EUROPEAN PATENT APPLICATION NO.**

EP 21 20 0432

This annex lists the patent family members relating to the patent documents cited in the above-mentioned European search report.
The members are as contained in the European Patent Office EDP file on
The European Patent Office is in no way liable for these particulars which are merely given for the purpose of information.

17-02-2022

| Patent document cited in search report | Publication date | Patent family member(s) | Publication date |
|---|---|---|---|
| WO 2021191886    A1 | 30-09-2021 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **O. TROTT ; A. J. OLSON.** AutoDock Vina: improving the speed and accuracy of docking with a new scoring function, efficient optimization and multithreading. *Journal of Computational Chemistry,* 2010, vol. 31, 455-461